# EUROPEAN PATENT SPECIFICATION

(11) **EP 2 018 208 B1**
(45) Date of publication and mention of the grant of the patent: **05.09.2012**
(21) Application number: 07783896.9
(22) Date of filing: 17.05.2007
(51) Int. Cl.: A61M 25/00, A61M 25/01, A61M 25/06

(54) **TRANSSEPTAL NEEDLE ASSEMBLIES AND METHODS**
TRANSSEPTALE NADELANORDNUNGEN UND VERFAHREN
ENSEMBLES AIGUILLES TRANSSEPTALES ET MÉTHODES

(30) Priority: 17.05.2006 US 800853 P; 29.12.2006 US 647312
(43) Date of publication of application: 28.01.2009
(73) Proprietor: St. Jude Medical, Atrial Fibrillation Division, Inc., St. Paul, Minnesota 55117-9913 (US)
(72) Inventor: HASSETT, James A., Eden Prairie, MN 55347 (US); STEHR, Richard E., Stillwater, MN 55082 (US)
(74) Representative: Allgayer, Ulla Barbara
(86) International application number: PCT/US2007/069180
(87) International publication number: WO 2007/137136

(56) References cited:
- DE-U1- 29 700 622
- US-A- 5 542 930
- US-A- 5 545 141
- US-A1- 2006 009 715
- US-E- R E34 086

## Description

### CROSS-REFERENCE TO RELATED APPLICATIONS

This application claims priority to United States provisional application no. 60/800,853, filed 17 May 2006 (the 853 application). This application also claims priority to United States nonprovisional application no. 11/647,312, filed 29 December 2006 (the '312 application), now pending.

### BACKGROUND OF THE INVENTION

### a. Field of the Invention

The instant invention relates to needle assemblies and methods for puncturing or piercing tissue within the body, including, for example, transseptal access systems and methods for accessing the left atrium from the right atrium by crossing the interatrial septum. In particular, embodiments of the instant invention are directed toward medical devices used with catheter assemblies in cardiology procedures that require transseptal puncture(s). The puncture assemblies have a moveable puncture device within a dilator being biased in a retracted position, the position of which is precisely locatable. The puncture assembly is preferably flexible along the majority of the length of the assembly, and therefore can be used with any catheter assembly of any predetermined shape, and adjustable from a predetermined position within the shaft to a position extending beyond the end of the dilator when necessary for use in transseptal procedures. An infusion cannula is known from DE 29700622 U1 and an apparatus and a method for forming a channel through a stomach wall are known from US 5,545,141 A.

The puncture device is preferably axially flexible, but longitudinally rigid when placed within the lumen of the dilator. A safety mechanism, such as a spring biased member, a clip spacer, or similar locking mechanism, is located at the proximal end of the assembly, preferably within an operable handle providing for extension of the puncture assembly beyond the distal tip of the dilator, only upon the affirmative action of a user. When the force exerted by the user is removed, the puncture assembly automatically retracts back into the initial position within the dilator. Thus, when not being used for purposes of puncturing the septum, the puncture device is maintained within the dilator thereby increasing the safety of transseptal procedures.

### b. Background Art

The human heart includes a right ventricle, a right atrium, left ventricle, and left atrium. The right atrium is in fluid communication with the superior vena cava and the inferior vena cava. The tricuspid valve separates the right atrium from the right ventricle. The right atrium is separated from the left atrium by a septum that includes a thin membrane known as the fossa ovalis.

A wide variety of diagnostic and therapeutic procedures have been developed in which a catheter is transluminally advanced within a guide sheath or over a guidewire into various chambers and across valves of the heart. The most difficult chamber of the heart to access with a catheter is the left atrium. Access to the left atrium through the pulmonary artery is not possible. Approaches from the left ventricle are difficult, may cause arrhythmias and may present difficulty in obtaining stable catheter positioning. Accordingly, one of the accepted methods of accessing the left atrium involves catheterization through the femoral or left subclavian vein into the right atrium, and subsequent penetration of the interatrial septum, the fossa ovalis, to gain entry to the left atrium. This procedure is commonly referred to as transseptal catheterization.

The objectives of left atrial access can be either diagnostic or therapeutic. One therapeutic use is electrophysiological intervention, e.g., left atrial ablation. Catheter ablation involves the placement of energy (typically RF) through a catheter, into various locations of the heart to eradicate inappropriate electrical pathways affecting the heart function. When these locations are in the left atrium, the catheter through which the RF energy is placed typically is itself placed through transseptal catheterization.

Despite clinical acceptance of a wide variety of procedures which require access to the left atrium, significant room for improvement remains in the actual access technique. A number of risks, in addition to the risks associated with normal heart catheterization, are inherent in transseptal catheterization. For example, a major risk present stems from the use of known transseptal devices, which typically have a puncture device, such as a needle and/or stylet, exposed externally from the dilator. The exposed nature of the puncture device renders adjustment of the assembly within the heart difficult, as it increases the risks of unanticipated puncture within the guide sheath during insertion, and detrimentally affects the maneuverability of the device to the appropriate point at the septum.

Known puncture assemblies typically have the distal portion of the puncture assembly exposed from the distal portion of the dilator. This configuration provides the puncture risks discussed above. Other assemblies with mechanisms to hold the puncture assembly within the dilator place a biasing mechanism at the distal tip of the assembly. Placing this mechanism at the distal tip presents additional problems with flexibility and maneuverability of the mechanism in operation. Thus, there is a need to provide a puncture assembly where the puncture device is safely maintained at a substantially fixed location within the dilator until the assembly is positioned at the puncture point of the septum, and further having a displacement mechanism located at the proximal end of the assembly. Such an improved structure greatly improves the overall functionality and safety of transseptal medical devices. Details of embodiments of this improved structure and related methods are described in more detail below.

### BRIEF SUMMARY OF THE INVENTION

The present invention provides for transseptal medical devices as further disclosed in claim 1, having improved safety and maneuverability features.

According to one embodiment of the present invention, a transseptal medical device is provided comprising an elongate tubular member, such as a dilator, having a proximal end and a distal end, a puncture device disposed within the dilator, and a displacement mechanism operably connected to the puncture device at a proximal end of the puncture device, whereby the displacement mechanism is capable of advancing a distal portion of the puncture device from an initial position within the dilator to a position external to the dilator when a force is exerted upon the displacement mechanism. When the force is removed from the displacement mechanism, the distal portion of the puncture device retracts to the initial position within the dilator. Preferably, the displacement mechanism is operably connected to the puncture device or the dilator such that operation of the displacement mechanism moves the dilator in a direction toward a proximate end of displacement mechanism or moves the puncture device in a direction toward the distal end of the assembly. The puncture device further includes at least one rigid section located at the distal portion of the puncture assembly and/or at the proximal portion of the puncture device and further has a flexible section intermediate the distal and proximate portions of the assembly.

The puncture device may have a length substantially equal to the length of the dilator, or substantially less than the length of the dilator yet still capable of being extended by the displacement mechanism to the position external to the dilator.

The puncture device may be comprised of a flexible polymer, a flexible metal, or any similar material known to those of skill in the art. The puncture assembly of the present invention may be flexible at a number of portions and may be comprised of a needle, or a curved needle.

The displacement mechanism of the medical device may further include a safety mechanism, such as a spring, a clip, or locking mechanism, operably connected to the puncture assembly. Preferably, the safety mechanism holds the puncture assembly within the dilator when the mechanism is in an unbiased, or locked position.

The dilator of the transseptal medical device may further comprise a dilator distal end and a dilator proximal end, the dilator distal end having a cross-sectional dimension smaller than a cross-sectional dimension of the dilator proximal end.

Methods for puncturing a septum of a patient's heart are further discussed comprising the following steps: introducing a puncture assembly contained within a dilator into an area of the heart proximate a target area of the septum; extending the puncture assembly to a position external to said dilator proximate the target area of the septum; puncturing the target area of the septum; and retracting the puncture assembly to a position within the dilator. The methods may further comprise the step of advancing the dilator through the target area of the septum before retracting the puncture assembly, and may further comprise the step of advancing the dilator through the target area of the septum after retracting the puncture assembly.

Additional methods contemplated include methods for making an extendible transseptal medical device comprising the following steps: providing a dilator having an inner lumen; providing a puncture assembly having a puncture device and a flexible portion attached to the puncture device within the inner lumen of the dilator; and operably connecting a displacement mechanism to the puncture assembly allowing for the puncture assembly to be extended from a first position within the dilator to a second position external to a distal end of the dilator upon exertion of a force upon the displacement mechanism, and automatically retracting the puncture assembly to the first position when the force is removed from the displacement mechanism.

The foregoing and other aspects, features, details, utilities, and advantages of the present invention will be apparent from reading the following description and claims, and from reviewing the accompanying drawings.

### BRIEF DESCRIPTION OF THE DRAWINGS

Fig. 1 depicts a transseptal medical device according to one embodiment of the present invention having an outer sheath being configured for a transseptal puncture procedure, an elongate tubular member within the outer sheath, and a puncture device within the elongate tubular member. The medical device further contains a displacement mechanism at the proximal end of the device operably connected to the puncture device.

Fig. 2 depicts a transseptal medical device according to one embodiment of the present invention showing four components of the device: (1) a pre-formed sheath for use with transseptal procedures; (2) a flexible elongate tubular member; (3) a flexible puncture device; and (4) a displacement mechanism attached to the flexible puncture device.

Fig. 3 depicts a flexible puncture device according to one embodiment of the present invention connected to a displacement mechanism, both for use with the transseptal medical device of the present invention.

Fig. 4 depicts a displacement mechanism according to one embodiment of the present invention operably connected to a puncture device and further connected to a dilator having an inner lumen for housing the puncture device.

Fig. 5 depicts the distal end of a transseptal medical device according to one embodiment of the present invention in a puncture configuration having a sheath, a dilator, and a puncture device.

Fig. 6 is a cross-sectional view of a transseptal medical device according to one embodiment of the present invention having a dilator, a flexible puncture device disposed therein, and a displacement mechanism.

Fig. 7 is a cross-sectional view of a transseptal medical device according to another embodiment of the present invention having a dilator, a flexible puncture device disposed therein, and a displacement mechanism. The puncture device of this embodiment has a distal rigid portion, a proximal rigid portion, and a flexible needle section intermediate the distal and proximal rigid portions.

Fig. 8 is a schematic diagram exemplifying a transseptal puncture procedure wherein a transseptal medical device according to one embodiment of the present invention is inserted through the left subclavian vein, traveling into the right atrium.

### DETAILED DESCRIPTION OF THE INVENTION

In general, the instant invention relates to transseptal access systems and methods for accessing the left atrium from the right atrium by crossing the interatrial septum. In particular, the instant invention is directed toward medical devices used with catheter assemblies in cardiology procedures that require transseptal puncture(s).

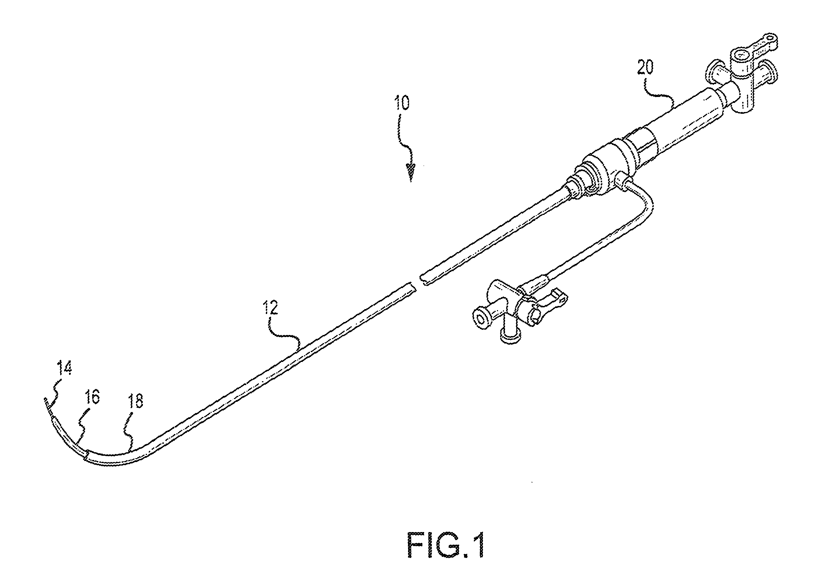

Fig. 1 shows an assembled puncture assembly 10 in accordance with one embodiment of the present invention, a portion of which is disposed within a sheath 12. The puncture assembly 10 has a retractable puncture device 14 disposed within a elongate tubular member, or for purposes of transseptal procedures, a dilator 16. The sheath 12 is preferably preconfigured with a bend 18 at an angle desirable for use with transseptal catheterization procedures. The puncture assembly 10 further includes a displacement mechanism 20 operably connected to either the puncture device 14 or the dilator 16, preferably the puncture device 14. In operation, the displacement mechanism 20 is operably connected to the puncture device 14 or the dilator 16 such that, when the displacement mechanism 20 is in an unbiased position, the distal end of the puncture device 14 is maintained at a predetermined, retracted position within the dilator 16 and the sheath 12. Upon exertion of a force upon the displacement mechanism 20, the puncture device 14 can be extended from an initial position within the dilator 16, to a distal position external to the dilator 16. This position is preferably the desired position for puncture of tissue in the body, e.g., the interatrial septum.

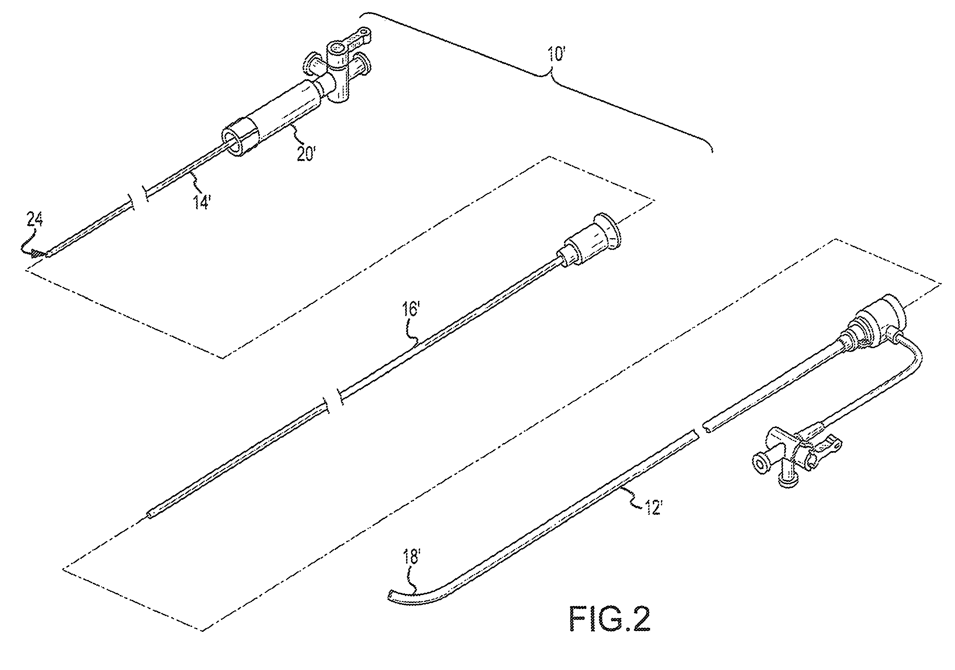

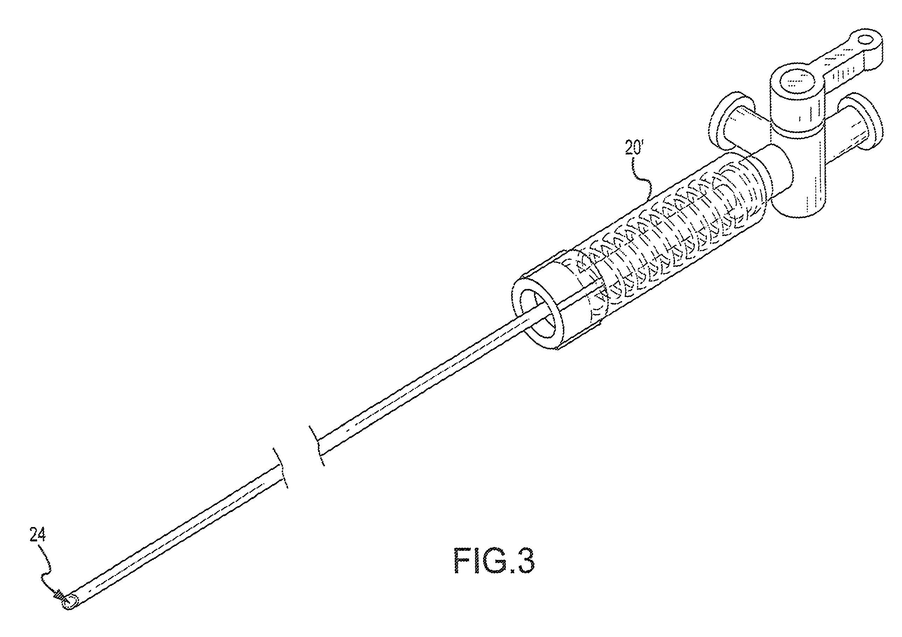

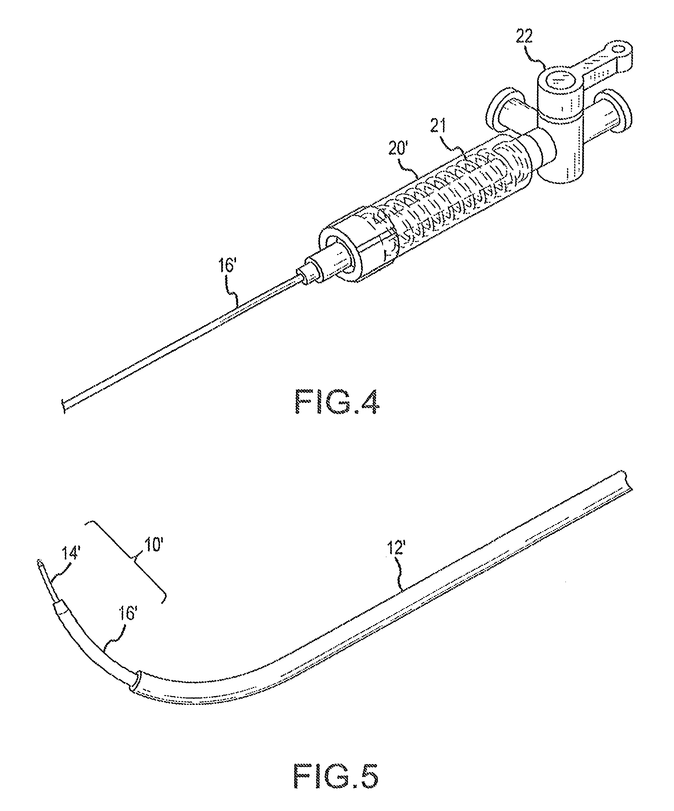

Fig. 2 shows various components of a flexible puncture assembly 10' and a sheath 12'. The flexible puncture assembly 10' includes a flexible puncture device 14', a displacement mechanism 20' operably connected to the puncture device 14', and a dilator 16'. The sheath 12' has a preformed bend 18' at its distal end and is configured to house both the dilator 16' and the puncture device 14'. Fig. 3 shows a flexible puncture device 14', operably connected to a displacement mechanism 20'. Fig. 4 identifies a displacement mechanism 20' operably connected to both a dilator 16' and a flexible puncture device 14' (not shown in Fig. 4) housed within the dilator and extending into the interior of the displacement mechanism 20'. A safety member or biasing member 21, e.g., a spring, a clip, or a locking mechanism, is disposed within the displacement mechanism 20'. Alternatively, a biasing mechanism 21 could be placed external to the distal end of the displacement mechanism 20'. Additionally, a valve 22 is operably connected to the puncture device 14' such that fluids can be delivered to, or removed from a target site through a lumen 24 (Figs 2 and 3) within the flexible puncture device 14'. The valve 22 further allows for insertion and retraction of medical devices, such as an ablation electrodes for performance of desired medical procedures.

Fig. 5 shows a side view of a puncture assembly 10', including a dilator 16' and a puncture device 14', disposed within a sheath 12'. The dilator 16' extends a portion beyond the distal end of the sheath 12'. The puncture device 14' is disposed within the dilator 16'. Preferably, the external diameter of the puncture device 14' closely approximates the inner diameter of the dilator 16' so as to provide axial rigidity to the puncture device 14'. This configuration allows the puncture device 14' to be made of any flexible material, such as a polymers, plastics, or flexible metal constructions. This configuration allows for flexibility of the puncture device 14' in axial or transverse directions to the longitudinal axis of the puncture device 14', while simultaneously allowing for structural rigidity necessary along the longitudinal axis for advancing and retracting the puncture device 14' within the dilator 16'.

Fig. 5 shows the puncture device 14' in the extended position, i.e., the position effected by exertion of a force by a user upon the displacement mechanism 20' (not shown in Fig. 5) operatively connected to the proximal end of the puncture assembly 10'. This position is preferable when the device is in position to pierce the tissue during a procedure. In the instance where there is no force acting on the displacement mechanism 20', the puncture device 14' is preferably at an initial, preset position within the dilator 16'. This preset, retracted position provides significant safety benefits over known puncture assemblies, where the puncture device remains exposed from the distal end of the dilator at virtually all times.

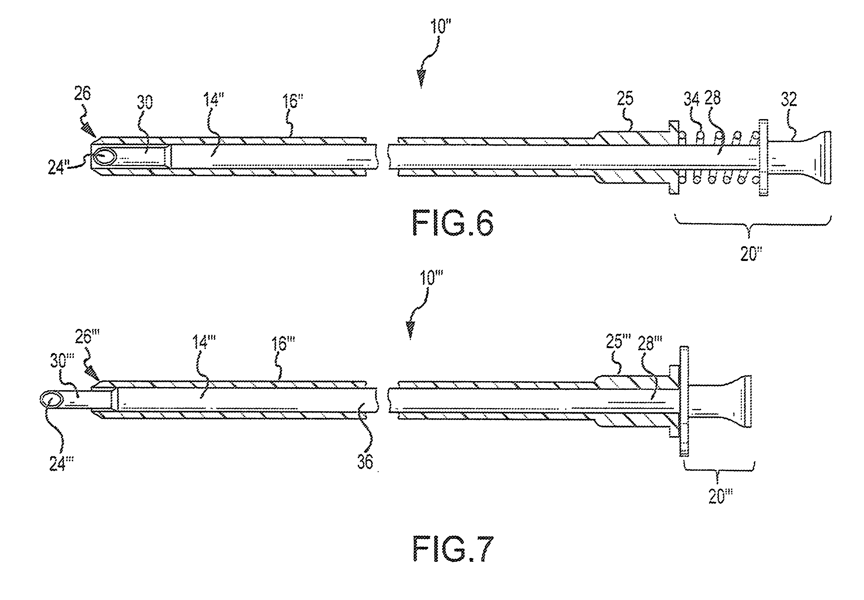

Fig. 6 is a cross-sectional view of a puncture assembly 10 "according to another embodiment of the present invention. The puncture assembly 10" includes a dilator 16" having a proximal end 25 and a distal end 26, the distal end 26 having a cross-sectional diameter less than the cross-sectional diameter of the proximal end 25. Disposed within an inner lumen of the dilator 16" is a flexible puncture device 14" having a proximal end 28 and a distal end 30. The distal end 30 of the flexible puncture device 14" has a cross-sectional diameter less than the cross-sectional diameter of the proximal end 28. The flexible puncture device 14" preferably has an inner lumen 24" for receiving a stylet (not shown). The stylet is removable from the inner lumen. The inner lumen 24" may also be used to deliver fluids to, or remove fluids from, a target site within the body.

The dilator 16" is preferably comprised of a flexible material, such as biocompatible polymers, plastics, braided wire assemblies, and combinations thereof, or any other suitable material known to those of skill in the art. This flexible construction allows for the use of the puncture assembly 10" with any known sheath (not shown) used for transseptal procedures.

The puncture device 14" is preferably flexible along the majority of the length of the assembly 10", and therefore can be used with any catheter assembly of any predetermined shape, and adjustable from a predetermined position within the shaft of the dilator 16" to a position extending beyond the distal end 26 of the dilator 16" when necessary for use in transseptal procedures. The puncture device 14" can be made of any flexible material such as polymers, plastics, flexible metal coils, or any other flexible material known to those of skill in the art. The puncture device 14" is preferably axially or transversally flexible, but longitudinally rigid when placed within the lumen of the dilator 16". In this regard, the lumen of the dilator 16" serves to contain the puncture device 14" in a configuration that is amenable to exertion of a longitudinal force for purposes of extension or retraction while maintaining axial flexibility.

The puncture assembly 10" further includes a displacement mechanism 20" connected to the proximal end 25 of the dilator 16" and/or the puncture device 14". The displacement mechanism 20" includes a proximal knob 32 for application of a force by a user. The displacement mechanism 20" further includes a safety member or biasing member 34, shown as a spring, configured to hold the puncture device 14" in an initial position where the distal end 30 of the puncture device 14" is within the distal portion 26 of the dilator 16". When a force is exerted on the displacement mechanism 20", the distal end 30 of the puncture device 14" extends a portion beyond the distal end 26 of the dilator 16". When the force exerted by the user is removed, the puncture device 14" automatically retracts back into the default biased position within the dilator 16". Thus, when not being used for purposes of puncturing tissue, the puncture device 14" is maintained within the dilator 16" thereby increasing the safety of procedures, such as transseptal procedures.

While the biasing member 34 is shown as a spring, it is contemplated that other structures can also be used, such as a retractable clip, a locking mechanism, or a screw mechanism, to safely contain the distal end 30 of the puncture device 14" at an initial position within the dilator 16", until exertion of a force by a user. Preferably, the mechanism 20" provides a restorative force to automatically retract the puncture device 14" upon removal of a force acting upon the knob 32. It is contemplated, however, that this retraction could also be implemented by a manual exertion of a force on the knob 32 in a direction away from the distal portion 26. In this instance, the safety mechanism 20" may be completely removed and/or replaced to prevent unwanted extension of the puncture device 14" to a position external to the distal portion 26 of the dilator 16".

Fig. 7 shows a cross-sectional view of the puncture assembly 10"' according to the present invention. The puncture device 14"' comprises a distal section 30"', a proximal section 28"', and an intermediate section 36. The distal 30'" and proximal section 28'" in this embodiment are preferably rigid sections with decreased flexibility. These sections may be made of any polymer, metal, or similar material known to those of skill in the art. The intermediate section 36 is preferably comprised of a flexible material such as polymers, plastics, flexible metal coils, or any other flexible material known to those of skill in the art. Preferably, the flexible intermediate section 36 tracks the curves of the dilator 16" and/or sheath (not shown in Fig. 7). The flexible intermediate section 36 provides for increased flexibility of the puncture device 14"' during use, while the rigid sections 28'" and 30"' provide for increased strength at the proximal and distal ends of the puncture device 14"' for applications where a more rigid puncture tip is desired, or where it is contemplated that additional force is necessary to pierce the targeted tissue area. It also provides increased ability to accurately locate and position the puncture assembly 14"' for a more precise transseptal crossing. The portions may be bonded or attached together by any number of manners well known to one of ordinary skill in the art. While the embodiment shown in Fig. 7 identifies three separate portions of the puncture device 14"', it is further contemplated that more portions are possible. It is contemplated that any combination of flexible and rigid portions may be provided depending on the desired combination of flexibility and rigidity of the transseptal device.

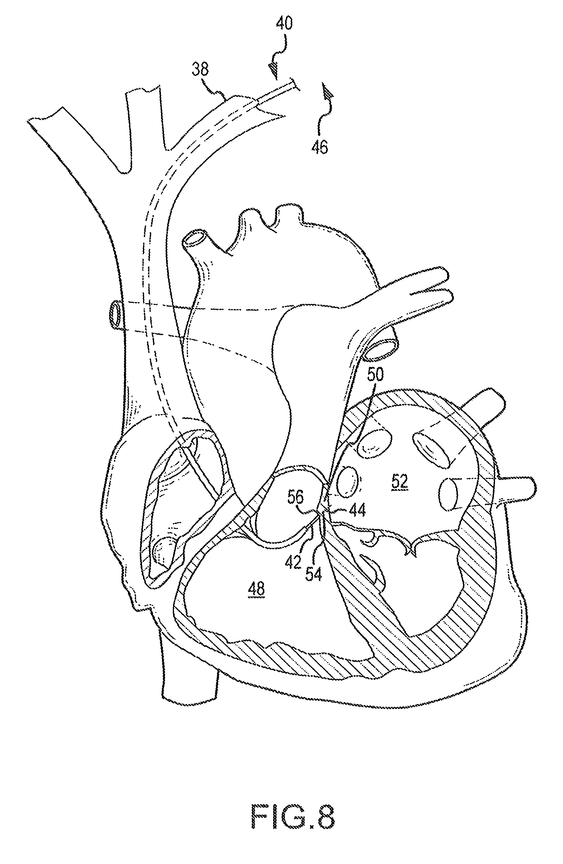

Fig. 8 shows a schematic diagram of a transseptal puncture procedure gaining access to the left atrium through the left subclavian vein 38. In this procedure, a transseptal medical device 40 according to one embodiment of the present invention is provided having a dilator 42, a puncture device 44, and a displacement mechanism (not shown in Fig. 8) located at the proximal end of the medical device 40. The medical device 40 is inserted through the left subclavian vein 38 and passed into the right atrium 48, where the device 40 is capable of performing a transseptal puncture allowing access to the left atrium 52 for further diagnostic or therapeutic treatment. The puncture device according to this embodiment comprises a flexible coil assembly (not shown) within the lumen of the dilator 42. The dilator 42 has a beveled distal end 54 for facilitation of the process. The puncture device 44 is shown in the extended position, i.e., with a force exerted upon the displacement mechanism. Thus, the puncture device 44 is in the position to puncture the interatrial septum 50.

Methods for puncturing a septum of a patient's heart are further discussed. The methods will be described in conjunction with the exemplary embodiment shown in Fig. 8. The methods preferably comprise the following steps: (1) introducing a puncture device 44 contained within a dilator 42 into an area of the heart proximate a target area 56 of the septum 50; (2) extending the puncture device 44 to a position external to the dilator 42 proximate the target area 56 of the septum 50; (3) puncturing the target area 56 of the septum 50; and (4) retracting the puncture device 44 to a position within the dilator 42. The methods further comprise the step of advancing the dilator 42 through the target area 56 of the septum 50 before retracting the puncture device 44, and optionally, advancing the dilator 42 through the target area 56 of the septum 50 after retracting the puncture device 44. In either instance, the result of the methods yield a conduit for delivery or removal of fluids or medical devices to any targeted area within the left atrium 52. This method therefore provides for safer access to the difficult to reach left atrium for the performance of medical procedures such as ablative, mappings, or other known procedures.

Additionally, the present invention contemplates methods for making an extendible transseptal medical device having increased safety features and beneficial maneuverability. The methods comprise the steps following: (1) providing a dilator having an inner lumen; (2) providing a puncture device having a flexible portion within the inner lumen of the dilator; and (3) operably connecting a displacement mechanism to the proximal end of the puncture device allowing for the puncture device to be extended from a first position within the dilator to a second position external to a distal end of the dilator upon exertion of a force upon the displacement mechanism. The method further contemplates automatically retracting the puncture device to the first position when the force is removed from the displacement mechanism.

Although a number of embodiments of this invention have been described above with a certain degree of particularity, those skilled in the art could make numerous alterations to the disclosed embodiments without departing from the spirit or scope of this invention.

All directional references (e.g., upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not limiting.

## Claims

1. A transseptal medical device (10, 10', 10", 10''') comprising
a dilator (16, 16', 16", 16"') having a proximal end and a distal end;
a puncture device (14, 14', 14", 14"') disposed within the dilator (16, 16', 16'', 16"'); and
a displacement mechanism (20, 20', 20", 20"') operably connected to the puncture device (14, 14', 14", 14"') at a proximal end of the puncture device (14, 14', 14", 14"'), whereby the displacement mechanism (20, 20', 20", 20"') is capable of advancing a distal portion of the puncture device (14, 14', 14", 14"') from an initial position within the dilator (16, 16', 16", 16''') to a position external to the dilator (16, 16', 16", 16"') when a force is exerted upon the displacement mechanism (20, 20', 20'', 20"'), and when the force is removed from the displacement mechanism (20, 20', 20", 20"'), the distal portion of the puncture device (14, 14', 14", 14"') retracts to the initial position within the dilator (16, 16', 16", 16"'),
**characterized in that** the puncture device (14, 14', 14", 14"') comprises a flexible section (36"') and a rigid section between the flexible section and the displacement mechanism.

2. The transseptal medical device (10, 10', 10", 10"') of claim 1, wherein the puncture device (14, 14', 14", 14"') is a length substantially equal to a length of the dilator (16, 16', 16", 16'").

3. The transseptal medical device (10, 10', 10", 10''') of claim 1, wherein the puncture device (14, 14', 14", 14"') is a length substantially less than a length of the dilator (16, 16', 16", 16"') yet still capable of being extended by the displacement mechanism (20, 20', 20", 20"') to the position external to the dilator (16, 16', 16", 16"').

4. The transseptal medical device (10, 10', 10", 10"') of any one of claims 1 to 3, wherein the puncture device (14, 14', 14", 14"') comprises a needle.

5. The transseptal medical device (10, 10', 10", 10"') of any one of claims 1 to 3, wherein the puncture device (14, 14', 14", 14"') comprises a curved needle.

6. The transseptal medical device (10, 10', 10'', 10''') of claim 1, wherein the dilator (16, 16', 16", 16"') further comprises a dilator distal end (26, 26"') and a dilator proximal end (25, 25"'), the dilator distal end (26, 26"') having a cross-sectional dimension smaller than a cross-sectional dimension of the dilator proximal end (25, 25'").

7. The transseptal medical device (10, 10', 10", 10''') of claim 1, wherein at least a portion of the puncture device (14, 14', 14", 14"') is further comprised of a flexible polymer.

8. The transseptal medical device (10, 10', 10", 10"') of claim 1, wherein at least a portion of the puncture device (14, 14', 14", 14"') is further comprised of a flexible metal.

9. The transseptal medical device (10, 10', 10'', 10"') of claim 1, wherein at least a portion of the puncture device (14, 14', 14", 14"') is further comprised of a flexible coil of a polymer, a metal, or a combination thereof.

10. The transseptal medical device (10, 10', 10", 10"') of claim 1, wherein the displacement mechanism is operably connected to the dilator (16, 16', 16", 16"'), such that operation of the displacement mechanism (20, 20', 20", 20"') moves the dilator (16, 16', 16", 16'") in a direction toward a proximate end of the displacement mechanism (20, 20', 20", 20"').

11. The transseptal medical device (10, 10', 10", 10'") of claim 1, wherein the displacement mechanism (20, 20', 20", 20"') further comprises a spring mechanism (34) operably connected to the puncture device (14, 14', 14", 14"').

12. The transseptal medical device (10, 10', 10", 10"') of claim 11, wherein the spring mechanism (34) holds the puncture assembly within the dilator (16, 16', 16", 16'") when the spring mechanism (34) is in an unbiased position.

13. The transseptal medical device (10, 10', 10", 10"') of claim 1 further comprising a mechanism to exert a retraction force on the displacement mechanism (20, 20', 20", 20"'), thereby retracting the puncture device to the position internal the dilator (16, 16', 16", 16"').

14. A method for making an extendible transseptal medical device (10, 10', 10", 10'') comprising the following steps:
providing a dilator (16, 16', 16", 16"') having an inner lumen;
providing a puncture device (14, 14', 14", 14"') having a flexible portion and a rigid portion located proximally of the flexible portion within the inner lumen of the dilator (16, 16', 16", 16"');
operably connecting a displacement mechanism (20, 20', 20", 20"') to the puncture device (14, 14', 14", 14"') proximally of the rigid portion of the puncture device (14, 14', 14", 14"') allowing for the puncture device (14, 14', 14", 14"') to be extended from a first position within the dilator (16, 16', 16", 16"') to a second position external to a distal end of the dilator (16, 16', 16", 16"') upon exertion of a force upon the displacement mechanism (20, 20', 20", 20"'), and automatically retracting the puncture device (14, 14', 14", 14"') to the first position when the force is removed from the displacement mechanism (20, 20', 20", 20"').

## Patentansprüche

1. Transseptale medizinische Vorrichtung (10, 10', 10", 10"'), enthaltend
einen Dilatator (16, 16', 16", 16"'), der ein nahes Ende und ein fernes Ende aufweist;
eine Punktiereinrichtung (14, 14', 14", 14"'), die innerhalb des Dilatators (16, 16', 16", 16"') angeordnet ist; und
einen Verschiebemechanismus (20, 20', 20", 20"'), der betriebsbereit mit der Punktiereinrichtung (14, 14', 14", 14"') an einem nahen Ende der Punktiereinrichtung (14, 14', 14", 14"') verbunden ist, wodurch der Verschiebemechanismus (20, 20', 20", 20"') geeignet ist, einen entfernten Bereich der Punktiereinrichtung (14, 14', 14", 14"') von einer Ausgangsposition innerhalb des Dilatators (16, 16', 16", 16"') zu einer Position außerhalb des Dilatators (16, 16', 16", 16"') zu bewegen, wenn eine Kraft auf den Verschiebemechanismus (20, 20', 20", 20"') aufgebracht wird, und, wenn die Kraft von dem Verschiebemechanismus (20, 20', 20", 20"') entfernt wird, sich der entfernte Bereich der Punktiereinrichtung (14, 14', 14", 14"') zu der Ausgangsposition innerhalb des Dilatators (16, 16', 16", 16"') zurück zieht,
**dadurch gekennzeichnet, dass** die Punktiereinrichtung (14, 14', 14", 14"') einen flexiblen Abschnitt (36"') und einen steifen Abschnitt zwischen dem flexiblen Abschnitt und dem Verschiebemechanismus (20, 20', 20", 20"') aufweist.

2. Transseptale medizinische Vorrichtung (10, 10', 10", 10"') nach Anspruch 1, wobei die Punktiereinrichtung (14, 14', 14", 14"') eine Länge im Wesentlichen gleich einer Länge des Dilatators (16, 16', 16", 16"') aufweist.

3. Transseptale medizinische Vorrichtung (10, 10', 10", 10"') nach Anspruch 1, wobei die Punktiereinrichtung (14, 14', 14", 14"') eine Länge im Wesentlichen geringer als einer Länge des Dilatators (16, 16', 16", 16"') aufweist, wobei die Punktiereinrichtung (14, 14', 14", 14"') immer noch geeignet ist, mittels des Verschiebemechanismus (20, 20', 20", 20"') zu der Position außerhalb des Dilatators (16, 16', 16", 16"') ausgefahren zu werden.

4. Transseptale medizinische Vorrichtung (10, 10', 10", 10"') nach einem der Ansprüche 1 bis 3, wobei die Punktiereinrichtung (14, 14', 14", 14"') eine Nadel aufweist.

5. Transseptale medizinische Vorrichtung (10, 10', 10", 10"') nach einem der Ansprüche 1 bis 3, wobei die Punktiereinrichtung (14, 14', 14", 14"') eine gebogene Nadel aufweist.

6. Transseptale medizinische Vorrichtung (10, 10', 10", 10"') nach Anspruch 1, wobei der Dilatator (16, 16', 16", 16"') ferner ein fernes Dilatatorende (26, 26"') und ein nahes Dilatatorende (25, 25"') aufweist, wobei das ferne Dilatatorende (26, 26"') eine Querschnittsabmessung aufweist, die kleiner als eine Querschnittsabmessung des nahen Dilatatorendes (25, 25"') ist.

7. Transseptale medizinische Vorrichtung (10, 10', 10", 10"') nach Anspruch 1, wobei wenigstens ein Bereich der Punktiereinrichtung (14, 14', 14", 14"') ferner aus einem flexiblen Polymer besteht.

8. Transseptale medizinische Vorrichtung (10, 10', 10", 10"') nach Anspruch 1, wobei wenigstens ein Bereich der Punktiereinrichtung (14, 14', 14", 14"') ferner aus einem flexiblen Metall besteht.

9. Transseptale medizinische Vorrichtung (10, 10', 10", 10"') nach Anspruch 1, wobei wenigstens ein Bereich der Punktiereinrichtung (14, 14', 14", 14"') ferner aus einer flexiblen Spule aus einem Polymer, einem Metall oder einer Kombination davon besteht.

10. Transseptale medizinische Vorrichtung (10, 10', 10", 10"') nach Anspruch 1, wobei der Verschiebemechanismus derart betriebsbereit mit dem Dilatator (16, 16', 16", 16"') verbunden ist, dass die Betätigung des Verschiebemechanismus (20, 20', 20", 20"') den Dilatator (16, 16', 16", 16"') in eine Richtung zu einem nahegelegenen Ende des Verschiebemechanismus (20, 20', 20", 20"') bewegt.

11. Transseptale medizinische Vorrichtung (10, 10', 10", 10"') nach Anspruch 1, wobei der Verschiebemechanismus (20, 20', 20", 20"') ferner einen Federmechanismus (34) aufweist, der betriebsbereit mit der Punktiereinrichtung (14, 14', 14", 14"') verbunden ist.

12. Transseptale medizinische Vorrichtung (10, 10', 10", 10"') nach Anspruch 11, wobei der Federmechanismus (34) die Punktierbaueinheit innerhalb des Dilatators (16, 16', 16", 16"') hält, wenn sich der Federmechanismus (34) an einer nicht-vorgespannten Position befindet.

13. Transseptale medizinische Vorrichtung (10, 10', 10", 10"') nach Anspruch 1, ferner enthaltend einen Mechanismus zum Aufbringen einer Rückzugskraft auf den Verschiebemechanismus (20, 20', 20", 20"'), wodurch die Punktiereinrichtung (14, 14', 14", 14"') zu der Position im Inneren des Dilatators (16, 16', 16", 16"') zurück gezogen wird.

14. Verfahren zum Herstellen einer ausfahrbaren transseptalen medizinischen Vorrichtung (10, 10', 10", 10"'), enthaltend die folgenden Schritte:
Bereitstellen eines Dilatators (16, 16', 16", 16"'), der einen Innenraum aufweist;
Bereitstellen einer Punktiereinrichtung (14, 14', 14", 14"'), die einen flexiblen Bereich und einen steifen Bereich, der nahe des flexiblen Bereichs im Inneren des Innenraums des Dilatators (16, 16', 16", 16"') angeordnet ist, aufweist;
Verbinden eines Verschiebemechanismus (20, 20', 20", 20"') betriebsbereit mit der Punktiereinrichtung (14, 14', 14", 14"') nahe des steifen Bereichs der Punktiereinrichtung (14, 14', 14", 14"'), so dass es der Punktiereinrichtung (14, 14', 14", 14"') ermöglicht wird, von einer ersten Position innerhalb des Dilatators (16, 16', 16", 16"') zu einer zweiten Position außerhalb eines entfernten Endes des Dilatators (16, 16', 16", 16"') durch Aufbringung einer Kraft auf den Verschiebemechanismus (20, 20', 20", 20"') ausgefahren zu werden, und die Punktiereinrichtung (14, 14', 14", 14"') sich automatisch zu der ersten Position zurück zieht, wenn die Kraft von dem Verschiebemechanismus (20, 20', 20", 20"') entfernt wird.

## Revendications

1. Dispositif médical transseptal (10, 10', 10", 10'") comprenant :
un dilatateur (16, 16', 16", 16'") ayant une extrémité proximale et une extrémité distale ;
un dispositif de perforation (14, 14', 14", 14"') disposé à l'intérieur du dilatateur (16, 16', 16", 16"') ; et
un mécanisme de déplacement (20, 20', 20", 20"') raccordé en service au dispositif de perforation (14, 14', 14", 14"') à une extrémité proximale du dispositif de perforation (14, 14', 14", 14"'), de sorte que le mécanisme de déplacement (20, 20', 20", 20"') soit à même de faire avancer une partie distale du dispositif de perforation (14, 14', 14", 14"') d'une position initiale à l'intérieur du dilatateur (16, 16', 16", 16"') à une position extérieure au dilatateur (16, 16', 16", 16"') lorsqu'une force est exercée sur le mécanisme de déplacement (20, 20', 20", 20"') et que, lorsque la force est retirée du mécanisme de déplacement (20, 20', 20", 20"'), la partie distale du dispositif de perforation (14, 14', 14", 14"') se retire dans la position initiale à l'intérieur du dilatateur (16, 16', 16", 16"'),
**caractérisé en ce que** le dispositif de perforation (14, 14', 14", 14"') comprend une section flexible (36"') et une section rigide entre la section flexible et le mécanisme de déplacement.

2. Dispositif médical transseptal (10, 10', 10", 10"') selon la revendication 1, dans lequel le dispositif de perforation (14, 14', 14", 14"') a une longueur sensiblement égale à la longueur du dilatateur (16, 16', 16", 16"').

3. Dispositif médical transseptal (10, 10', 10", 10"') selon la revendication 1, dans lequel le dispositif de perforation (14, 14', 14", 14"') a une longueur sensiblement inférieure à la longueur du dilatateur (16, 16', 16", 16"'), mais est encore capable de s'étendre sous l'effet du mécanisme de déplacement (20, 20', 20", 20"') dans la position extérieure au dilatateur (16, 16', 16", 16"').

4. Dispositif médical transseptal (10, 10', 10", 10"') selon l'une quelconque des revendications 1 à 3, dans lequel le dispositif de perforation (14, 14', 14", 14"') comprend une aiguille.

5. Dispositif médical transseptal (10, 10', 10", 10"') selon l'une quelconque des revendications 1 à 3, dans lequel le dispositif de perforation (14, 14', 14", 14"') comprend une aiguille incurvée.

6. Dispositif médical transseptal (10, 10', 10", 10"') selon la revendication 1, dans lequel le dilatateur (16, 16', 16", 16"') comprend en outre une extrémité distale (26, 26"') et une extrémité proximale (25, 26"'), l'extrémité distale (26, 26"') du dilatateur ayant une dimension en coupe transversale plus petite que la dimension en coupe transversale de l'extrémité proximale (25, 25"') du dilatateur.

7. Dispositif médical transseptal (10, 10', 10", 10"') selon la revendication 1, dans lequel au moins une partie du dispositif de perforation (14, 14', 14", 14"') est en outre constituée d'un polymère flexible.

8. Dispositif médical transseptal (10, 10', 10", 10"') selon la revendication 1, dans lequel au moins une partie du dispositif de perforation (14, 14', 14", 14"') est en outre constituée d'un métal flexible.

9. Dispositif médical transseptal (10, 10', 10", 10"') selon la revendication 1, dans lequel au moins une partie du dispositif de perforation (14, 14', 14", 14"') est en outre constituée d'un enroulement flexible d'un polymère, d'un métal ou d'une de leurs combinaisons.

10. Dispositif médical transseptal (10, 10', 10", 10"') selon la revendication 1, dans lequel le mécanisme de déplacement est raccordé en service au dilatateur (16, 16', 16", 16"') de sorte que le fonctionnement du mécanisme de déplacement (20, 20', 20", 20"') déplace le dilatateur (16, 16', 16", 16"') dans une direction vers une extrémité proximale du mécanisme de déplacement (20, 20', 20", 20"').

11. Dispositif médical transseptal (10, 10', 10", 10"') selon la revendication 1, dans lequel le mécanisme de déplacement (20, 20', 20", 20"') comprend en outre un mécanisme à ressort (34) raccordé en service au dispositif de perforation (14, 14', 14", 14"').

12. Dispositif médical transseptal (10, 10', 10", 10"') selon la revendication 11, dans lequel le mécanisme à ressort (34) maintient l'ensemble de perforation dans le dilatateur (16, 16', 16", 16"') lorsque le mécanisme à ressort (34) se trouve en position non sollicitée.

13. Dispositif médical transseptal (10, 10', 10", 10"') selon la revendication 1, comprenant en outre un mécanisme pour exercer une force de retrait sur le mécanisme de déplacement (20, 20', 20", 20"'), rétractant de la sorte le dispositif de perforation dans la position intérieure au dilatateur (16, 16', 16", 16"').

14. Procédé de fabrication d'un dispositif médical transseptal extensible (10, 10', 10", 10"'), comprenant les étapes suivantes consistant à :
mettre en oeuvre un dilatateur (16, 16', 16", 16"') ayant une lumière interne ;
mettre en oeuvre un dispositif de perforation (14, 14', 14", 14"') ayant une partie flexible et une partie rigide située en position proximale par rapport à la partie flexible dans la lumière interne du dilatateur (16, 16', 16", 16"') ;
raccorder en service un mécanisme de déplacement (20, 20', 20", 20"') au dispositif de perforation (14, 14', 14", 14"') en position proximale par rapport à la partie rigide du dispositif de perforation (14, 14', 14", 14"') pour permettre au dispositif de perforation (14, 14', 14", 14") de s'étendre d'une première position à l'intérieur du dilatateur (16, 16', 16", 16"') à une seconde position extérieure à une extrémité distale du dilatateur (16, 16', 16", 16"') en exerçant une force sur le mécanisme de déplacement (20, 20', 20", 20'"), et de retirer automatiquement le dispositif de perforation (14, 14', 14", 14"') dans la première position lorsque la force est retirée du mécanisme de déplacement (20, 20', 20", 20"').
